# EUROPEAN PATENT SPECIFICATION

(11) **EP 0 544 837 B1**
(45) Date of publication and mention of the grant of the patent: **05.11.1997**
(21) Application number: 91919977.8
(22) Date of filing: 07.08.1991
(51) Int. Cl.: A61B 5/00, A61B 5/14

(54) **DISPOSABLE SKIN PERFORATOR AND BLOOD TESTING DEVICE**
EINWEG-VORRICHTUNG ZUM DURCHSTECKEN DER HAUT UND ZUR BLUT-UNTERSUCHUNG
PERFORATEUR DERMIQUE JETABLE ET DISPOSITIF ANALYSEUR DU SANG

(30) Priority: 10.08.1990 US 566160; 18.07.1991 US 732109
(43) Date of publication of application: 09.06.1993
(73) Proprietor: SWIERCZEK, Remi, Concord, OH 44077 (US)
(72) Inventor: SWIERCZEK, Remi, Concord, OH 44077 (US)
(74) Representative: Feakins, Graham Allan
(86) International application number: PCT/US91/05616
(87) International publication number: WO 92/02175

(56) References cited:
- EP-A- 0 189 117
- WO-A-85/04089
- WO-A-88/09149
- WO-A-91/08712
- DE-A- 3 515 420
- US-A- 4 648 408
- US-A- 4 994 068

## Description

This invention, generally, relates to a device for drawing a small amount of blood from a person's fingertip for analysis, and more particularly to a single use device for obtaining a blood sample employing a means for preventing multiple use of the device.

Blood testing is a common practice. The samples can be derived by merely pricking the fingertip with a sharp tool. Then, the samples must be exposed to proper test medium to acquire the test result. In the past, a complex, sudden release, pen type device with disposable blades was used to perforate the skin painlessly. Once the skin was cut, separate test medium was introduced to the blood sample.

A disposable device for collecting blood is known, for example, from the lancet shown and described in US-A-4 677 979.

According to the present invention, there is provided a skin perforator for puncturing the skin, said perforator comprising a housing having a contact surface constituting a pressure plate for placement adjacent to the skin to be punctured, an aperture in said contact surface, and a puncturing member mounted within said housing for movement towards and through said aperture, upon the application of applied force said puncturing member penetrating into the skin, characterised by a shutter means for occluding the aperture of said skin perforator following initial puncture of the skin.

An embodiment can include a test medium attached to a pressure plate on the device which can absorb blood flowing from the puncture.

Another embodiment provides a convenient holding means for litmus paper and other type test medium in such a way to allow for saturation of the test medium with blood while it is drawn.

Yet another embodiment provides a package that consists of a chart and bandage. The chart can be colour coded to read the test results while the bandage can be used to protect the cut finger.

The skin perforator can possess a self-contained means for cleansing and/or disinfecting the puncture site, prior to perforation of the skin.

For a better understanding of the invention and to show how the same may be carried into effect, reference will now be made, by way of example, to the accompanying drawings, in which:-
Figure 1 is a plan view of a skin perforator illustrating a shutter means for preventing multiple use, in its tensioned state,
Figure 2 is a cross-sectional view of the skin perforator in Figure 1, taken across line 2-2,
Figure 3 is a plan view of the skin perforator shown in Figures 1 and 2, illustrating the shutter means in its untensioned state,
Figure 4 is a cross-sectional view of the skin perforator shown in Figure 3, taken along line 4-4,
Figure 5 is a plan view of a skin perforator illustrating an alternative shutter means for preventing multiple use in its tensioned state,
Figure 6 is a cross-sectional view of the skin perforator shown in Figure 5 taken along line 6-6,
Figure 7 is a plan view of the skin perforator shown in Figures 5 and 6 and illustrating the shutter means in its untensioned state,
Figure 8 is a cross-sectional view of the skin perforator as shown in Figure 7 taken along line 8-8,
Figure 9 is a plan view of a convex dome for a skin perforator having an integrally formed shutter means,
Figure 10 is an underneath view of another embodiment of a skin perforator illustrating a self-contained means for cleansing and/or disinfecting the puncture site, and
Figure 11 is a cross-sectional view of skin perforator illustrating a removable guard incorporated therein for maintaining the sterility of barb until use.

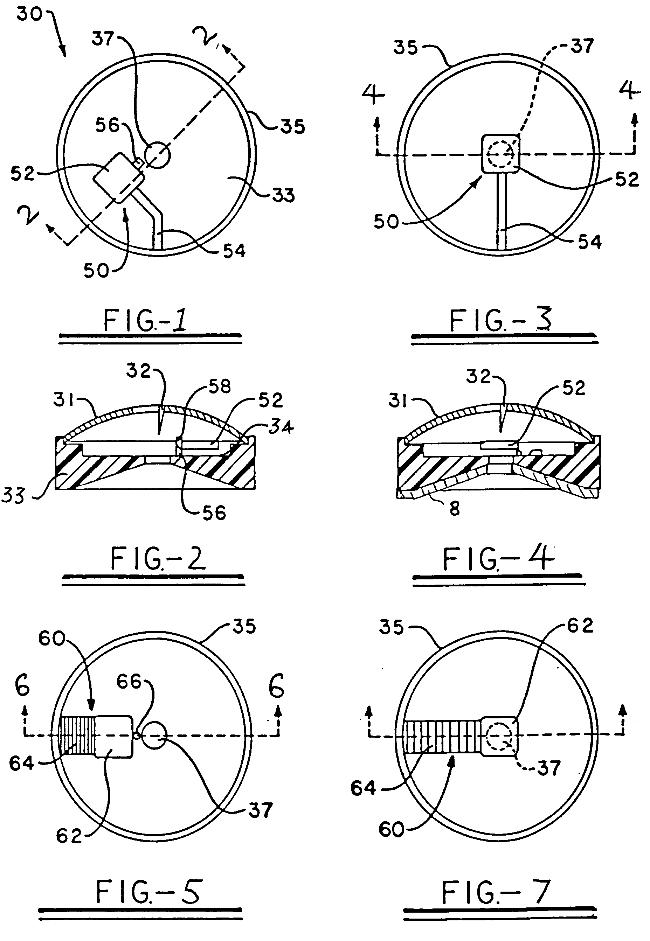

Figures 1 to 4 disclose a preferred embodiment 30 of the skin perforator. A pressure plate 33 is preferably made from a moulded plastics material and is essentially annular in shape. A resilient convex panel 31 having a point penetrating barb 32, is seated on a step 38 and inside of an outer rim 35 in the pressure plate 33. The pressure plate 33 contains an aperture 37 in its centre which allows passage of the barb 32 therethrough. Shelf 34 serves to stop the inversion of the panel 31, thereby controlling the depth of the barb 32 into the patient's skin.

In order to achieve a quick and relatively painless penetration of the skin, distance D between the peak of the panel 31, in its undepressed state, and shelf 34 of pressure plate 33 is preferably at least about .10 inches (2.54 mm). While this distance is not absolute, it has been calculated to account for a minimum stroke depth, deflection waste and differences in patient skin texture.

According to the present embodiments, the minimum depth stroke is identified as that distance necessary for the panel to undergo its inversion and is estimated at .050 inches (1.27 mm). The deflection waste refers to the movement of the panel 31 towards the puncture site upon the application of force but prior to the panel's inversion and is calculated as about .025 inches (0.635 mm). Approximately .030 inches (0.762 mm) is necessary to account for differences in the thickness and texture of the skin at the puncture site. A distance D ranging from about 0.075 inches (1.905 mm) to about 1.025 inches (26.035 mm) ensures penetration of the skin coupled with a positive return of the inverted panel to its convex configuration.

The present device 30 is designed to perforate the skin on the finger of the patient. The finger should be prepared so as to cleanse and remove as many contaminates from the perforation site as is possible using techniques which are well known in the art.

Having applicability to all embodiments, the device 30 is operated by positioning the device 30 against the patient's finger to be punctured so that the intended puncture site of the finger comes within an area defined by the outer circumference of the aperture 37 in the pressure plate 33. Pressure is then applied to the panel by the patient or person assisting the same. The initial applied pressure causes the pressure plate 33 to be pressed against the skin of the patient further isolating the intended puncture site. As additional pressure is applied to the panel 31 the stress accumulation in the panel will result in the sudden inward collapsing of the panel 31, directing the barb 32 through the aperture 37, thereby causing a piercing of the patient's skin. This inward movement of the barb 32 is stopped as the inner surface of the panel 31 strikes the shelf 34 of the pressure plate 3 to provide a predictable puncture depth. As the applied pressure on the panel 31 and pressure plate 33 is removed or reduced, the collapsed panel will resiliently return to its original shape and accordingly withdraw the barb 32 from the patient's finger.

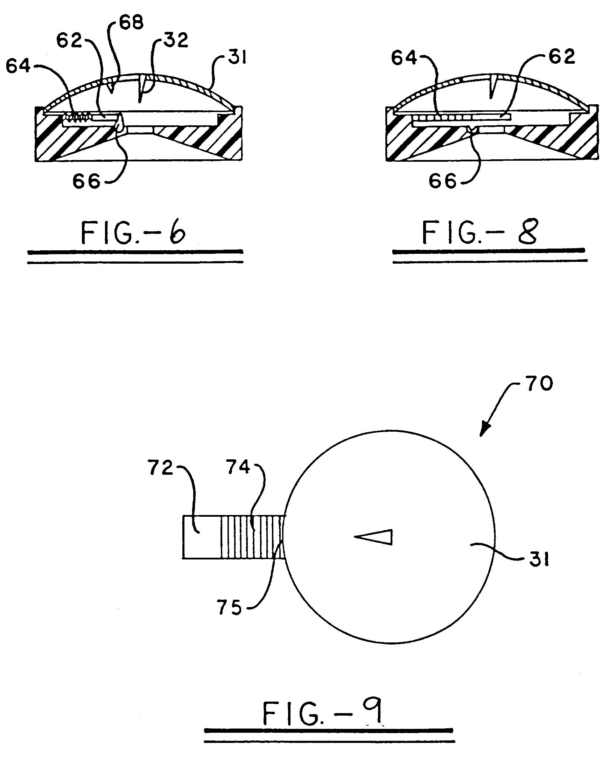

The device, in a further preferred embodiment, comprises an absorbent test strip 8 and is best illustrated in the cross-sectional views of Figure 4. The test strip 8 is disclosed as generally a pad or sheet of material capable of absorbing blood or exudate from the puncture site created by barb 32. The test strip 8 is contemplated as being treated or coated with various reagents which react with the blood or exudate to cause colour or chemical changes which then can be quantitatively or qualitatively compared to a known standard. Most home use test strips utilise colour change techniques and yield fairly quick results which can be interpreted by comparing the test strip to a colour coded chart provided with the device. The most common test strip contemplated is that used for the determination of the patient's blood sugar level. The technology of such test strips is well known in the art.

The test strip 8 is disclosed as being laminated to the outer surface of the pressure plate 33. Lamination may be done using known adhesives. The test strip 8 may be of varying shapes or sizes but preferably has a length slightly greater than the diameter of aperture 37. Although, not required, the test strip 8 contains an aperture dimensioned and positioned to allow passage of barb 32 therethrough.

Following the puncture of the patient's finger by the barb 32, the disposable skin perforator 30 remains in contact with the finger to allow the blood or exudate from the puncture site to be absorbed onto test strip 8. Maintenance of a pressure pressing pressure plate 33 against the finger, but which is insufficient to cause collapse of the panel 31, will enhance blood flow as previously described and allow for saturation of the test strip 8 with the blood or exudate from the puncture site.

This device, incorporating the test strip 8 places the test medium immediately adjacent to the puncture site and allows blood collection and testing to begin immediately upon puncture. Furthermore, the test strips 8 representing various thickness may be used. Such strips 8 can extend into the recession found in the bottom surface of pressure plate 33 by shelf 38. In a less preferred, but contemplated embodiment, test strip 8 would omit the formation of aperture therein. The barb 32 is of sufficient length and sharpness to cut test strip 8 simultaneously with the skin of the fingertip. However, it is thought that such a practice may permit the introduction of fibrous material from test strip 8 into the puncture site formed by the barb 32.

The skin perforators of the present invention are intended as disposable or single use devices. In this way, the risk of patient contamination resulting from the use of unsterile or tainted devices is minimised. In furtherance of this objective, the present invention provides for a shutter means for preventing repeated use of the device, as illustrated in the Figures.

Generally, the device is fully functional upon its removal from the aseptic packaging. The device is used to perforate the skin of a patient for the purposes of obtaining a blood sample by placing the pressure plate in contact with the skin and applying force to the convex panel. The force applied to cause inversion of the panel resulting in the puncture of the panel by the barb 32 is also utilised to fracture or otherwise disable a stop member functioning to hold a shutter device in its tensioned position. Upon retraction of the panel, the shutter means will advance to its untensioned position between the barb 32 and aperture 37. In this way, subsequent attempts to utilise the device 30 as a skin perforator will force the barb 32 against the shutter, preventing puncture of the patient's skin. Once the shutter means has advanced to its untensioned position between barb 32 and aperture 37, the device is disabled and to be discarded.

Specifically, Figures 1-4 illustrate one embodiment of a shutter means for preventing repeated use of the skin perforator. Figures 1 and 3 disclose the shutter means generally 50, having a shield portion 52 and a stem portion 54. The stem 54 is contemplated as an essentially rigid material such as to return to its original untensioned position even after extended periods of time in a tensioned state. Such suitable materials include but are not limited to various types of plastics or aluminium. The proximal end of stem 54 is shown as attached to an inner wall of the pressure plate 33 although it is to be recognised that alternative sites of attachment are possible.

A shield member 52 is secured to the distal end of the stem 54. The shield member 52 is intended as having a total area approximately equal or greater to the aperture 37.

A stop member 56 is located between the aperture 37 and shutter means 50 and serves to maintain the shutter means 50 in a tensioned position until completion of the device's initial use. The step member 56 is intended to be constructed with sufficient strength to enable it to hold the shutter means 50 in a tensioned position but capable of fracturing, deforming, pivoting or otherwise yielding to the force of the skin perforator's activating mechanism, in this case the panel, in order to release the shutter means thereby preventing further use of this particular device.

The stop member 56 is contemplated as being made from material similar to the pressure plate 33 and formed integrally therewith. A frangible connection or weakened portion 58 is formed therein but possessing sufficient strength to withstand the pressure of a tensioned shutter means 50. Upon compression of the panel 31 from applied force, the frangible connection 58 is broken as the barb penetrates the skin. Upon retraction of the panel 31 to its original position, the shutter means 50 advances to its untensioned position as illustrated in Figures 3 and 4 with the shield 52 covering the aperture 37. In this way, subsequent attempts to utilise device 30 will result in the barb's contact with the shield 52 thereby preventing additional punctures of the skin using the same device.

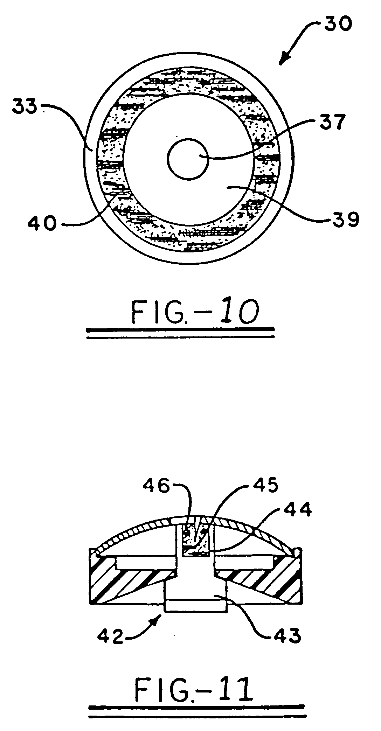

Figures 5-8 illustrate an alternative shutter means 60 utilising the same general principles previously discussed. A shutter stem 64 is secured to an inner wall of the pressure plate 33 by any means known in the art and is formed from any corrugated material capable of returning to an elongate position following compression. A stop member 66 is disclosed as a raised projection originating from the surface 34 and formed integrally therefrom. The stop member 66 is compressed into a void or weakened region of the pressure plate 33 by the force of the panel 31 as it directs the barb 32 into the skin.

The shutter means as illustrated in the embodiments previously disclosed herein need only possess sufficient tension to enable it to position itself between the barb and the aperture. Preferably the tension applied to the shutter means is not so great as to cause contact with the barb, while in the patient's skin. Alternatively, a retaining means 68 as shown in Figure 6 may be formed in or on panel 31 to delay the advance of the shutter means to its untensioned position until after the barb is withdrawn from the puncture site.

Figure 9 shows a panel 31 and shutter means 70 formed as a single unit from resilient metal or plastics materials. One such embodiment is contemplated as being formed from the stamping of a resilient metal material as described previously with respect to the panel 1.

The shutter means 70 is folded towards the interior of the panel 31 along the line 75. During assembly, the stem 74 is compressed in order that the shutter 70 is retained in a tensioned state until actuated by a stop member.

Optionally, any of the embodiments disclosed herein are contemplated as capable of incorporating a self-contained cleaning means disclosed in Figure 10. Device 30 is shown as incorporating an absorbent pad, impregnated with a cleansing and/or disinfecting agent, affixed to the lower surface 39 of the pressure plate 33, adjacent the patient's skin.

The absorbent pad 40 is intended to be made from any cotton, synthetic or other fibre commonly used in the art for such purpose and capable of retaining liquid therein. The cleansing or disinfecting agents can be selected from any such agents known in the art, including but certainly not limited to ethyl alcohol, isopropyl alcohol or benzalkonium chloride.

The lower surface 39 of the pressure plate 33 is aseptically covered or sealed to keep the surface free from contaminates and to prevent evaporation of the cleansing agent. Immediately prior to use, a packaging seal is removed thus exposing the device and/or lower surface 39. The eventual puncture site can then be cleansed or disinfected by rubbing the lower surface 39 of the device 30 over the site. Often times, the puncture site is a fingertip, at which time the device is simply rubbed between the thumb and fingertip. In this manner, the eventual puncture site is cleansed and prepared to receive the point penetrating barb.

Figure 11 reveals a means for preserving the sterility of the barb 32 until just prior to the time of desired use. This packing means is disclosed as a tab 42 having a base portion 43 and a stem 44. The stem 44 is formed with a cavity 45 open at the end opposite base 43. The cavity 45 has a depth at least equal to the length of barb 32 in the panel 31. The cavity 45 is capable of receiving a wax or other medium exhibiting properties of a solid at or above room temperature but liquefying in the presence of heat. Additionally, such medium must possess the ability to prevent or resist the growth of bacteria, fungi and other microbes, either inherently or by the addition of antimicrobials.

Following the sterilisation of the barb 32 after assembly, the cavity 45 in the stem 44 is filled with a medium 46 in a liquid or semi-solid state. Immediately thereafter, the barb 32 is inserted into the medium contained in the cavity 45. Thereupon cooling, a frangible connection between the tab 42 and the barb 32 is formed. The tab 42 is removed prior to use of the skin perforator by pliably moving the tab 42 relative to the device 30. Other means of frangibly connecting the tab 42 about the barb 32 as are known in the art to accomplish the same or similar purpose, are contemplated, such as ultrasonic welding and the like.

The shutter means detailed in the drawings and specification of this application are illustrative and not to be construed as limited to the specific embodiments set forth herein. It is contemplated that the self-contained cleansing means and the shutter means have application well beyond the scope of the specific embodiments of skin perforators disclosed in this application, the scope of the application in its whole being only limited by the claims.

Hence the foregoing embodiments are designed to be simple in construction, economical to manufacture and capable of being packaged under sterile conditions.

## Claims

1. A skin perforator for puncturing the skin, said perforator comprising a housing having a contact surface constituting a pressure plate for placement adjacent to the skin to be punctured, an aperture (37) in said contact surface, and a puncturing member (32) mounted within said housing for movement towards and through said aperture, upon the application of applied force said puncturing member penetrating into the skin, characterised by a shutter means (50) for occluding the aperture of said skin perforator following initial puncture of the skin.

2. A perforator according to claim 1, wherein said shutter means comprises a shield portion (52) dimensioned approximately equal to or greater than the aperture (37) in said pressure plate, and a stem portion (54) capable of accepting applied tension and so as to advance predictably upon removal of said tension.

3. A perforator according to claim 1 or 2, wherein said skin perforator further comprises a stop member (56) for retaining said shutter in a tensioned position.

4. A perforator (20) according to any one of the preceding claims and comprising a cover plate (21) having a resilient convex panel (1) formed therein, said cover plate being attached to said pressure plate and said convex panel having a point penetrating barb (2), constituting said puncturing member, formed integrally therefrom and directed inward toward said aperture in said pressure plate, wherein a pressure applied to the convex panel and directed toward said pressure plate overcomes the resistance of said convex panel and said convex panel undergoes at least a partial inversion to cause said barb to suddenly and rapidly penetrate the skin causing a puncture and said convex panel resiliently returning to its original convex shape upon removal of said pressure thereby withdrawing the barb from the skin.

5. A perforator according to claims 3 and 4, wherein said stop member has sufficient strength to retain said shutter in a tensioned position but yields to a force applied during the conversion of said convex panel so as to release the tension applied to said shutter.

6. A perforator according to claim 5, wherein said shutter means is activated by the inversion of the convexity of said convex panel.

7. A perforator according to claim 4, 5 or 6, wherein said perforator further comprises a means (34) for controlling the penetration depth of said skin by said barb.

8. A perforator according to any one of claims 4 to 7, wherein said perforator further comprises a pad (40) attached to said pressure plate to absorb blood or exudate flowing from a puncture site formed by the penetration of said barb.

9. A perforator according to claim 8, wherein said pad is impregnated with a skin preparatory agent comprising one or more from the group consisting of ethyl alcohol, isopropyl alcohol, benzalkonium chloride, iodine, and topical antimicrobials.

10. A perforator according to claim 8 or 9, wherein said pad is a substantially annular ring circumscribing the aperture in said pressure plate.

11. A perforator according to claim 8, 9 or 10, wherein said pad is treated with at least one reagent for medical analysis.

## Patentansprüche

1. Ein Hautdurchstecher zum Einstechen in die Haut, wobei der Durchstecher ein Gehäuse mit einer Kontaktfläche, die eine Druckplatte zur Anordnung der zu durchstechenden Haut bildet, eine Öffnung (37) in der Kontaktfläche und ein Einstechteil (32) aufweist, welches innerhalb des Gehäuses für eine Bewegung in Richtung und durch die Öffnung hindurch angeordnet ist, wobei bei der Aufbringung einer anliegenden Kraft das Einstechteil in die Haut einsticht, gekennzeichnet durch eine Verschlußvorrichtung (50) zum Verschließen der Öffnung in dem Hautdurchstecher nach dem ersten Einstechen in die Haut.

2. Ein Durchstecher nach Anspruch 1, wobei die Verschlußvorrichtung einen Abschirmabschnitt (52) mit einer Abmessung annähernd gleich oder größer als die Öffnung (37) in der Druckplatte und einen Stielabschnitt (54) aufweist, der in der Lage ist, eine anliegende Spannung derart aufzunehmen, daß er sich bei Abbau der Spannung vorhersagbar bewegt.

3. Ein Durchstecher nach Anspruch 1 oder 2, wobei der Hautdurchstecher weiterhin ein Anschlagteil (56) aufweist, um den Verschluß an einer gespannten Position zu halten.

4. Ein Durchstecher (30) nach einem der vorhergehenden Ansprüche mit einer Abdeckplatte (33), in der eine elastisch nachgiebige konvexe dünne Platte (31) ausgebildet ist, wobei die Abdeckplatte an der Druckplatte angebracht ist und die konvexe dünne Platte einen punktförmig durchstechenden Dorn (32) hat, der das Einstechteil bildet, hieran einstückig ausgebildet ist und nach innen in Richtung der Öffnung der Druckplatte weist, wobei ein auf die dünne Platte aufgebrachter und in Richtung der Druckplatte verlaufender Druck den Widerstand der konvexen dünnen Platte überwindet und die konvexe dünne Platte zumindest eine teilweise Formumkehrung erfährt, um zu bewirken, daß der Dorn plötzlich und schnell die Haut durchsticht und einen punktförmigen Einstich bewirkt und wobei die konvexe dünne Platte bei Entfernung des Druckes elastisch/nachgiebig in ihre ursprüngliche konvexe Form zurückkehrt, so daß der Dorn aus der Haut zurückgezogen wird.

5. Ein Durchstecher nach Anspruch 3 und 4, wobei das Anschlagteil eine ausreichende Festigkeit hat, um den Verschluß in einem gespannten Zustand zu halten, jedoch unter einer während der Formumkehrung der konvexen dünnen Platte aufgebrachte Kraft nachzugeben, um die am Verschluß anliegende Spannung abzubauen.

6. Ein Durchstecher nach Anspruch 5, wobei die Verschlußvorrichtung bei der Formumkehrung der Konvexität der konvexen dünnen Platte aktiviert wird.

7. Ein Durchstecher nach Anspruch 4, 5 oder 6, wobei der Durchstecher weiterhin eine Vorrichtung (34) zur Steuerung der Eindringtiefe des Dornes in die Haut aufweist.

8. Ein Durchstecher nach einem der Ansprüche 4 bis 7, wobei der Durchstecher weiterhin ein Kissen (40) aufweist, das an der Druckplatte angebracht ist, um Blut oder Exsudat aufzunehmen, das aus der Einstichstelle ausfließt, welche durch das Einstechen des Dorns gebildet wurde.

9. Ein Durchstecher nach Anspruch 8, wobei das Kissen mit einem Hautbehandlungsmittel aus einem oder mehreren Mitteln der Gruppe Ethylalkohol, Isopropylalkohol, Benzalkoniumchlorid, Jod und topische keimtötende Mittel getränkt ist.

10. Ein Durchstecher nach Anspruch 8 oder 9, wobei das Kissen ein im wesentlichen kreisförmiger Ring ist, der die Öffnung in der Druckplatte umgibt.

11. Ein Durchstecher nach Anspruch 8, 9 oder 10, wobei das Kissen mit wenigstens einem Reagenz für medizinische Analysen behandelt ist.

## Revendications

1. Un perforateur dermique pour percer la peau, ledit perforateur comprenant une enveloppe ayant une surface de contact constituant une plaque d'appui pour être placée contre la peau à perforer, une ouverture (37) dans ladite surface de contact, et un élément de perforation (32) monté à l'intérieur de ladite enveloppe pour se mouvoir vers et à travers ladite ouverture, ledit élément de perforation pénétrant dans la peau lors de l'application d'une force, caractérisé par un dispositif d'obturation (50) pour fermer l'ouverture dudit perforateur dermique à la suite de la perforation initiale de la peau.

2. Un perforateur selon la revendication 1, dans lequel ledit dispositif d'obturation comprend une partie écran (52) de dimension approximativement égale ou supérieure à l'ouverture (37) dans ladite plaque d'appui, et une partie tige (54) capable d'accepter la tension appliquée et de telle façon qu'elle avance de manière prévisible lorsque ladite tension est relâchée.

3. Un perforateur selon la revendication 1 ou 2, dans lequel ledit perforateur dermique comprend également un élément d'arrêt (56) pour maintenir ledit obturateur dans une position de tension.

4. Un perforateur (30) selon l'une ou l'autre des revendications précédentes et comprenant une plaque de couverture (33 ) ayant un panneau convexe élastique (31) formé dedans, ladite plaque de couverture étant fixée à ladite plaque d'appui et ledit panneau convexe ayant une pointe pénétrante (32) constituant ledit élément de perforation, intégralement formée à partir du panneau et orientée vers l'intérieur, vers ladite ouverture dans ladite plaque d'appui, dans lequel une pression appliquée au panneau convexe et orientée vers ladite plaque d'appui a raison de la résistance dudit panneau convexe et ledit panneau convexe subit au moins une inversion partielle pour faire en sorte que ladite pointe pénètre brusquement et rapidement la peau provoquant une perforation et ledit panneau convexe revenant par résilience à sa forme convexe d'origine lorsque ladite pression est relâchée, retirant ainsi la pointe de la peau.

5. Un perforateur selon les revendications 3 et 4, dans lequel ledit élément d'arrêt a suffisamment de force pour maintenir ledit obturateur dans une position de tension, mais cède à une force appliquée pendant l'inversion dudit panneau convexe de manière à relâcher la tension appliquée audit obturateur.

6. Un perforateur selon la revendication 5, dans lequel ledit dispositif d'obturation est activé par l'inversion de la convexité dudit panneau convexe.

7. Un perforateur selon la revendication 4, 5 ou 6, dans lequel ledit perforateur comprend aussi un dispositif (34) pour contrôler la profondeur de pénétration de ladite peau par ladite pointe.

8. Un perforateur selon l'une ou l'autre des revendications 4 à 7, dans lequel ledit perforateur comprend aussi un tampon (40) fixé à ladite plaque d'appui pour absorber le sang ou exsudat coulant du site de perforation formé par la pénétration de ladite pointe.

9. Un perforateur selon la revendication 8, dans lequel ledit tampon est imprégné d'un agent de préparation de la peau comprenant l'un ou plusieurs des agents du groupe constitué par l'alcool éthylique, l'isopropyle alcool, le benzalkonium chlorure, l'iode et les antimicrobiens topiques.

10. Un perforateur selon la revendication 8 ou 9, dans lequel ledit tampon est substantiellement une rondelle annulaire circonscrivant l'ouverture dans ladite plaque d'appui.

11. Un perforateur selon la revendication 8, 9 ou 10, dans lequel ledit tampon est traité avec au moins un réactif pour analyses médicales.
